# EUROPEAN PATENT SPECIFICATION

(11) **EP 0 632 034 B1**
(45) Date of publication and mention of the grant of the patent: **19.08.1998**
(21) Application number: 94107917.0
(22) Date of filing: 24.05.1994
(51) Int. Cl.: C07D 401/06, C07D 417/06, A01N 51/00, A01N 43/78, A01N 43/647, A01N 43/40

(54) **Agents for preserving technical materials against insects**
Mittel zur Präservierung von technischer Materialien gegen Insekten
Agents pour préserver des matériaux techniques contre les insectes

(30) Priority: 04.06.1993 JP 158201/93
(43) Date of publication of application: 04.01.1995
(73) Proprietor: NIHON BAYER AGROCHEM K.K., Tokyo 108 (JP)
(72) Inventor: Tsuboi, Shin-ichi, Shimotsuga-gun, Tochigi (JP)
(74) Representative: Linkenheil, Dieter

(56) References cited:
- EP-A- 0 386 565
- EP-A- 0 428 941
- EP-A- 0 483 055
- WO-A-93/25080

## Description

The present invention relates to the use of known nitroimino compounds as agents for combating technical materials destroying insects in order preserve these materials.

The resent invention also relates to compositions useful for completely, i.e. not only against insects but also against fungi, bacteria and algae and for treating soil to protect technical materials against termite infestations.

The invention furthermore relates to processes for treating technical materials and for soil treatment against termite infestations.

The compounds and their insecticidal use in the field of plant protection has already been known. Compare for example with Japanese Laid-open patent application No. 235881/1990.

Insecticidal agents and compositions of said compounds and their use to preserve technical materials completely and to treat soil against termite infestations have not been known up to now.

Deferent insects are known as pests infesting technical materials so that due to serious damages caused thereby undesirable effects on living environment and cultural assets principally made of these materials have posed a social problem, urgently requiring effective controlling of the pests. Termites are known as important examples of pests.

At present, use for combating technical materials destroying insects has been made of organophosphorus insecticides such as phoxim [O-(α-cyanobenzylideneamino) O,O-diethylphosphoro-thioate], chloropyriphos [O,O-diethyl-3,5,6-trichloro-2-pyridylphosphorothioate], etc., as well as pyrethroides series insecticides such as permethrin [5-benzyl-3-furymethyl-3-(2-methoxy-carbonyl-1-propenyl)-2,2-dimethylcyclopropane carboxylate], decamethrin [α-cyano-3-phenoxybenzyl *d,l*-cis-3-(2,2-dibromovinyl)-2,2-dimethyl cyclopropane carboxylate], cypermethrin [α-cyano-3-phenoxybenzyl (±) *cis, trans*-3-(2,2-dichlorovinyl)-2,2-dimethylcyclopropane-carboxylate], fenvalerate [(RS)-α-cyano-3-phenoxybenzyl (RS)-2-(4-chlorophenyl)-3-methylbutyrate], cyfluthrine [cyano-(4-fluoro-3-phenoxy-phenyl) methyl-3-(2,2-dichloroethenyl)-2,2-dimethyl-cyclopropane carboxylate].

However, the above-mentioned insecticides are unsatisfactory as far as effective concentration and the long lasting effect are concerned.

It has been found that the known compounds of the formula (I) 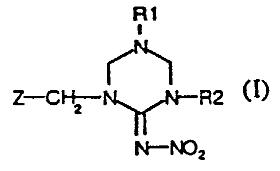 wherein Z represents 2-chloro-5-pyridiyl or 2-chloro-5-thiazolyl, and R1 and R2 each independently represents hydrogen or C₁₋₄ alkyl, can be used to preserve wood or composite wood materials against attack from wood destroying insects from the order isoptera and/ or coleoptera.

The compounds according to the invention of the formula (I) surprisingly exhibit an extremely strong insecticidal action on wood or composite wood destroying insects and the function is substantially superior to that of known insecticidal agents.

The compounds of the formula (I), the individual residues have the following preferable meanings: .
Z represents 2-chloro-5-pyridiyl or 2-chloro-5-thiazolyl, and
R1 and R2 represent methyl.

As examples of the active substance to be used according to the invention, the following ones are particularly preferred:
1-(6-chloro-3-pyridylmethyl)-3,5-dimethyl-2-nitroiminohexahydro-1,3,5-triazine,
1-(2-chlor-5-thiazolylmethyl)-3,5-dimethyl-2-nitroiminohexahydro-1,3,5-triazine,
1-(6-chloro-3-pyridylmethyl)-3-methyl-2-nitroiminohexahydro-1,3,5-triazine, and
1-(2-chloro-5-thiazolylmethyl)-3-methyl-2-nitroiminohexahydro-1,3,5-triazine.

The active substances to be used according to the invention can be used in insecticidal agents for combating wood or composite wood destroying insects and preserving these materials. They can also be used for soil treatment against termite infestation.

As individual examples of technical materials preserved by means of the insecticidal agents according to the present invention the following ones can be mentioned: wood or composite wood-materials (such as pressed wood, particle board, chip board , wafer board, plywood, wood laminated material, freshly cut timber/lumber etc.),

As individual examples of insects to be combated or controlled by the active substance of formula (I) according to the present invention the following ones can be mentioned:
Order Isoptera
   - Mastotermitidae Kalotermitidae: such as Kalotermes spp.
   Cryptotermes spp. etc.
   - Termopsidae: such as Zootermopsis spp. etc.
   - Rhinotermitidae: such as Reticulitermes spp.
   Heterotermes spp.
   Coptotermes spp.
   - Termitidae: such as Amitermes spp.
   Nasutitermes spp.
   Acanthotermes spp.
   Mikrotermes spp. etc.
Order Coleoptera
   - Siricidae: such as Sirex spp.
   Urocerus spp.
   - Formicidae: such as Camponotus spp.

In isoptera, as examples of termites, the following species are mentioned:
*Deucotermes speratus,*
*Coptotermes formosanus,*
*Glyptotermes fucus,*
*Glyptoterms satsumensis,*
*Glyptotermes nakajimai*
*Glyptotermes kodamai,*
*Incisitermes minor,*
*Neotennes koshunensis,*
*Crytotermes domesticus,*
*Hodotermopsis japonica,*
*Reticulitermes formosanus,*
*Nasutitermes takasagoensis,*
*Capritermes nitobei,* etc.

- Lyctidae: such as Lyctus brunneus etc.
- Bostrychidae: such as Bostrychus capucinus
Dinoderus minutus etc.
- Anobiidae: such as nobium punctatum
Xyletinus peltatus
Xestobium rufovillosum
Ptilinus pectinicomis etc.
- Cerambydidae: such as Hylotrupes bajulus Hesperophanus cinereus Srtomatium fulvum Chlorophorus pilosus etc.

Oedemeridae
Serropulpidae
Curculionidae
Seolytidae
Olatypodidae
Order Hymenoptera

The active compounds can be converted into the customary formulations, such as solutions, emulsions, wettable powders, suspensions, powders, foams, pastes, granules, tablets, aerosols, natural and synthetic materials impregnated with active compound, and microcapsules.

These formulations may be produced in known manner, for example by mixing the active compounds with extenders, that is to say liquid or liquefied gaseous or solid diluents or carriers, optionally with the use ofsurface-active agents, that is to say emulsifying agent sand/or dispersing agents and/or foam-forming agents. In the case of the use of water as an extender, organic solvents can, for example, also be used as auxiliary solvents.

As liquid solvents diluents or carriers, there are suitable in the main, aromatic hydrocarbons, such as xylene, toluene or alkyl napthalenes, chlorinated aromaticor chlorinated aliphatic hydrocarbons, such as chloro-benzenes, chloroethylenes or methylene chloride, aliphatic hydrocarbons, such as cyclohexane or paraffins, for example mineral oil fractions, alcohols, such as butanol or glycol as well as their ethers and esters, ketones, such as acetone, methyl ethyl ketone, methylisobutyl ketone or cyclohexanone, or strongly polar solvents, such as dimethylformamide and dimethyl-sulphoxide, as well as water.

By liquefied gaseous diluents or carriers are meant liquids which would be gaseous at normal temperature and under normal pressure, for example aerosol propellants, such as halogenated hydrocarbons as well as butane, propane, nitrogen and carbon dioxide.

As solid carriers there may be used ground natural minerals, such as kaolins, clays, talc, chalk, quartz, attapulgite, montmorillonite or diatomaceous earth, andground synthetic minerals, such as highly-dispersed silicic acid, alumina and silicates. As solid carriers for granules there may be used crushed and fractionated natural rocks such as calcite, marble, pumice, sepioliteand dolomite, as well as synthetic granules of inorganicand organic meals, and granules of organic material suchas sawdust, coconut shells, maize cobs and tobacco stalks.

As emulsifying and/or foam-forming agents there may beused non-ionic and anionic emulsifiers, such as polyoxyethylene-fatty acid esters, polyoxyethylene-fattyalcohol ethers, for example alkylaryl polyglycol ethers, alkyl sulphonates, alkyl sulphates, aryl sulphonates as well as albumin hydrolysis products.

Dispersing agents include, for example, lignin sulphite waste liquors and methylcellulose.

Adhesives such as carboxymethylcellulose and natural and synthetic polymers in the form of powders, granules or lattices, such as gum, polyvinyl alcohol and polyvinyl acetate, can be used in the formulation.

It is possible to use colorants such as inorganic pigments, for example iron oxide, titanium oxide and Prussian Blue, and organic dyestuffs, such as alizarin dye stuffs, azo dye stuffs or metal phthalocyanine dye stuffs, and trace nutrients, such as salts of iron, manganese boron, copper, cobalt, molybdenum and zinc.

The formulations in general contain from 0.001 to 95 percent by weight of active compound, preferably from 0.5 to 90 per cent by weight.

Furthermore, the active compound of the present invention having the formula (I) can be present as a mixture with a synergist useful. The term "synergist" denotes a compound which is not active in itself, but promotes the action of an active compound. The content of the active compounds having the general formula (I) of the present invention in commercially useful formulations can vary within a wide range. The active compound concentrations can very within a wide range. The active compound concentration of the formulation for use is, for example, from 0.0000001 to 100 present by weight, preferably from 0.0001 to 1 percent by weight.

In order to protect the above-mentioned materials completely, i.e. not only against material destroying insects but also against fungi, bacterial and algae, they can be treated with compositions containing at least one insecticidally active compound of the formula (I) and at least one biological active fungicide, bactericide or algizide.

Wood or composite wood-materials can be preferably be treated with a composition containing
a) an insecticidally effective amount of a compound of the formula (I) or mixtures thereof and
b) a fungicidally effective amount of at least one compound selected from the group of
   Trihalosulfenyl-Compounds such as
      N-Dichlorofluoromethylthio-N,N'-dimethyl-N-phenyl-sulfuric acid diamide (Dichlofluanide)
      N-Dichlorofluoromethylthio-N,N'-dimethyl-N-p-toluylsulphamide (Tolylfluanide)
      N-Trichloromethylthiophthalimide (Folpet)
      N-Dichlorofluoromethylthiophiophthalimide (Fluorfolpet) etc.
   Iodine-Compounds such as
      3-Iodo-2-propynyl-butylcarbamate
      3-Iodo-2-propynyl-hexylcarbamate
      3-Iodo-2-propynyl-cyclohexylcarbamate
      3-Iodo-2-propynyl-phenylcarbamate
      Diiodomethyl-p-tolylsulphone (Amical 48) etc.
   Phenols such as
      ortho-Phenylphenol
      Tribromophenol
      Tetrachlorophenol
      Pentachlorophenol etc.
   Azole-Compounds such as
      1-(4-Chlorophenoxy)-3,3-dimethyl-1-(1H-1,2,4 triazol -1-yl)-2-butanone (Triadimefon)
      β-(4-Chlorophenoxy)-α-(1,1-dimethyl-ethyl)-1H-1,2,4-triazole-1-ethanol (Triadimenol)
      ±α[2-(4-chlorophenyl)ethyl]-α-(1,1-dimethylethyl)-1H-1,2,4-triazole-1-ethanol (Tebconazol)
      1-[2(2,4-dichlorophenyl) 4-propyl-1,3-dioxolan-2-ylmethyl]-1H-1,2,4-triazole (Propiconazole)
      1-[2(2,4-dichlorophenyl)-1,3-dioxolan-2-ylmethyl]-1H-1,2,4-triazole (Azaconazol)
      (RS)-2(2,4-dichlorophenyl)-1-(1H-1,2,4 traizol-2-yl)-2ol (Hexaconazol)
      1-N-propyl-N-[2-(2,4,6-trichlorophenoxy)ethyl]carbamoylimidazol (prochloraz) etc.
   Tin Compounds such as
      Tributyl tin octuate
      Tributyl tin oleate
      Bistributyl tin oxide
      Tirbutyl tin naphthenate
      Tributyl tin phosphate
      Tributyl tin benzoate etc.,
   Thiocyanate Compounds such as
      Methylenebisthiocyanate (MBT)
      2-Thiocyanomethylthiobenzothiazole (TCMTB) etc,
   Quarternavy Ammonium Compounds such as
      Benzyl-dimethyl-tetradecylammoniumchloride
      Benzyl-dimethyl-dodecylaminonimchloride etc.
   Benzimidazole Compounds such as
      2-(2'-Fulyl)-1H-benzimidazole (Fuberidazole)
      Methylbenzimidazol-2-ylcarbamate (BCM)
      2-(4'-thiazolyl) benzimidazole (Thiabendazole)
      Methyl (1-butylcarbamoyl)-2benzimidazole carbanate (Benomyl)
   Isothiazolinone Compounds such as
      N-Methylisothiazolin-3-one
      5-Chloro-N-methylisothiazoline-3-one
      4,5-Dichloro-N-octylisothiazolin-3-one
      N-Octhylisothiazoline-3-one
   Morpholine Compounds such as
      C₁₄-C₁₁-4-Alkyl-2,6-dimethylmorpholine (Tridemorph)
   Pyridine Compounds such as
      1-Hydroxy-2-pyridine-thione and Sodium Iron, Manganese or Zinc-Salt thereof
      Tetrachloro-4-methyl sulphonyl pyridine
   N-Cyclohexyldiaziniumdioxy Compounds such as
      Tris-(N-cyclohexyldiaziniumdioxy) aluminium
      Bis-(N-cyclohexyldiaziniumdioxy)copper
   Naphthenate Compounds such as
      Zincnaphthenate
   Quinoline Compound such as
      8-hydroxy-quinoline
   Nitriles such as
      1,2,3,5-Tetrachloro-4,6-cyanobenzene.

These fungicidally effective compounds are added to the composition in order to prevent wood or wood materials not only against wood destroying insects but also against
Wood-discoloring fungi such as
Ascomycetes (*Caratocystis minor*)
Deuteromycetes (*Aspergillus niger*, *Aureobasidium pullulaus*, *Dactyleum fusarioides*, *Penicillium Vaeiable*, *Sclerophoma pithyophila*, *Scopularia phycomyces*, *Trichoderma viride*, *Trichoderma liguorum*)
Zygomycetes (*Mucor spinosus*)
and/or
Wood-destroying fungi such as
Ascomycetes (*Chetomium alba-arenulum*, *Chaetonium globosum*, *Humicola grisea*, *Petriella setifera*, *Trichurus speralis*)
Basidiamycetes (*Coniophera puteana*, *Coriolus versicolor*, *Donbiopora expansas*, *Glenospora graphii*, *Gloeophyllum abietinum*, *Gloeophyllum adoratum*, *Gloeophyllum protactum*, *Gloeophyllum trabeum*, *Gloeophyllum sepiarium*, *Lentinus cyathioformes*, *Lentinus edodes*, *Lentinus lepideus*, *Lentinus squavrolosus*, *Paxillus panuoides*, *Pleurofus*, *Poria placenta*, *Poria monticola*, *Poria vaillantii*, *Poria vaporia*, *Serpula himantoides*, *Serpula lacrymans*, *Tyromyces palustris*)
Deuteromycetes (*Cladosporium herbarum*).

Generally the compositions also will include at least one additional diluent, emulsifier, melting agent, organic binding agent, auxiliary solvents, processing additives, fixatives, plasticizers, UV-stabilizers or stability enhances, dyes (water soluble, water insoluble), color pigments, siccatives, corrosion inhibitors, anti settlement agents, additional insecticides (such as insecticidal carbamates, organophosphorus compounds, halogenoted hydrocarbons, pyrethroides etc.), anti skinning agents and the like.

The above-mentioned additional ingredients and their use are described in prior art. (EP-A 0370665, DE-A 3531257, DE-A 3414244).

The composition as used in the present invention generally comprise from 10⁻⁶ to 30 parts by weight, preferably from about 0.0005 to 15 parts by weight and more preferably from 0.005 to 2 parts by weight of the insecticide of the formula (I) and from 0.01 to 90 parts by weight, preferably from about 0.05 to 50 parts by weight and more preferably from 0.1 to 30 parts by weight of at least one on the above-mentioned fungicides.

The compositions can be provided as ready for use products or as concentrates, will have to be diluted prior to use.

The compositions can be applied by means of brushing, spraying, clipping, double vacuum and the like as known in the art. The compositions can be prepared by any technique known in the art.

The content of the present inventions will be concretely explained by way of the following examples but the present invention should not be limited only thereto.

Examples for compositions:

### Example 1

| | |
|---|---|
| 0.005% | 1-(6-chloro-3-pyridylmethyl)-3,5--dimethyl-2-nitroiminohexahydro-1,3,5-triazine (Compound I) |
| 5% | Butylglycol |
| 94.995% | Mineral spirits |

### Example 2 Impregnating agent/Primer

| | |
|---|---|
| 0.01% | Compound I |
| 0.5% | Dichlorofluanide |
| 1% | Tebconazole |
| 9.7% | Alkyd resin (solid) |
| 88.79% | Mineral spirits |

### Example 3 Wood stain/low build

| | |
|---|---|
| 0.01% | 1-(2-chloro-5-pyridylmethyl)-3,5-dimethyl-2-nitroiminohexahydro-1,3,5-triazine (Compound II) |
| 0.5% | Dichlorofluanide |
| 1.2% | Tebconazole |
| 21% | Alkyd resin (solid) |
| 2% | Pigment |
| 4% | Antisettlement additive, drys etc. |
| 71.29% | Mineral spirits |

### Example 4 Wood stain/high build

| | |
|---|---|
| 0.015% | Compound II |
| 0.6% | Dichlorofluanide |
| 1.5% | Tebconazole |
| 40% | Alkyd resin (solid) |
| 2% | Pigment |
| 4% | Antisettlement additive, drys etc. |
| 48.115% | Mineral spirits |

### Example 5 Soil treatment

| | |
|---|---|
| 20% | Compound I |
| 8% | ethylene glycol |
| 3% | emulsifiers |
| 0.25% | thickeners |
| 68.75% | distilled water |

### Example 6 Wood brushing

| | |
|---|---|
| 0.1% | Compound I |
| 1% | 3-bromo-2,3-diiodo-2-propenyl ethylcarbonate |
| 98.9% | organic solvents |

### Example 7 Wood brushing

| | |
|---|---|
| 0.1% | Compound II |
| 1.5% | 4-chlorophennyl-3-iodopropargylformal |
| 98.4% | organic solvents |

### Example 8 Formicidal tests

Compounds under test Examples of the active compounds according to the present invention
   Compound I
   Compound II
Comparative compounds
   A: phoxim
   B : chlorpyriphos

### Preparation of test formulation:

- solvent:: 3 parts by weight of xylene
- Emulsifier:: 1 parts by weight of poloxyethlene-alkylphenyl-ether

To prepare a suitable formulation of the active compound, I part by weight of each of the active compounds was mixed with the above-mentioned amount of the solvent containing the a mentioned amount of the emulsifier, and the mixture was diluted with water to a predetermined concentration.

### Test method:

1 ml of the aqueous solution prepared in the above-mentioned procedure was uniformly applied using a pipette onto a filter paper that was placed in petri dish of 9 cm diameter. Ten head of worker termites (*Copotermes formosanus*) were replaced into the petri dish and it was kept in a constant temperature chamber at 25 °C.

After four days, the mortality of the termites was investigated. This test procedure was carried out in duplicate per each concentration of the active compounds under test. The teat results are shown in Table 1.

**Table 1**

| Compound | Concentration of active compound (ppm) | Mortality of termites after four days (%) |
|---|---|---|
| I | 40. | 100 |
| | 8. | 100 |
| | 1.6 | 100 |
| | 0.32 | 100 |
| II | 40. | 100 |
| | 8. | 100 |
| | 1.6 | 100 |
| | 0.32 | 100 |
| Comparison A | 40. | 100 |
| | 8. | 100 |
| | 1.6 | 100 |
| | 0.32 | 90 |
| Comparison B | 40. | 100 |
| | 8. | 100 |
| | 1.6 | 100 |
| | 0.32 | 100 |
| Untreated | | 0 |

### Example 9 Test on Residual Effect

Small blocks of Japanese redpine tree (2 cm x 2 cm x 2 cm) were soaked for one minute into the aqueous solution prepared by the similar procedure to Example 8.

After air-dried, they were kept in a constant temperature camber at 40°C for four weeks. Then each of the thus of treated blocks was placed in a polymeric cup (10 cm diameter) containing 150 ml of sandy loam of 20 % moisture content. Into each of the polymeric cup, 100 head of working termites and 10 head of soldier termites (*Coptotermes formosanus*) were released. After three weeks, the degree of xylophagous damage in the block and the mortality of the termites were investigated.

Three test were carried out in duplicate 25 °C, and the results are shown in Table 2.

The index of xylophagous damage observed on the blocks:
- 0 :: No damage
- 0.5 :: One to two traces of damages each having a depth of about I mm form the block surface
- 1 :: One to two evident damages or more the one deep trace of damage having a depth of more than 2 mm from the block surface
- 2 :: More than three evident damages or more than one deep trace of damage having a depth of more than 2 mm from the block surface
- 3 :: More than three deep damages
- 4 :: Evidently damaged zone covering up to about one third of the whole surface area of the block
- 5 :: Evidently damaged zone covering more than one third of the wholesurface area of the block.

**Table 2**

| Compound | Concentration of active compound ( ppm ) | Mortality of termites after three weeks (%) | Degree of xylophagous damage in the pine tree block (0 - 5) |
|---|---|---|---|
| I | 40 | 100 | 0 |
| | 8 | 100 | 0 |
| | 1.6 | 100 | 0 |
| | 0.32 | 90 | 1 |
| I | 40 | 100 | 0 |
| | 8 | 100 | 0 |
| | 1.6 | 100 | 0 |
| | 0.32 | 85 | 2 |
| A | 40 | 25 | 3 |
| | 8 | 0 | 5 |
| | 1.6 | 0 | 5 |
| | 0.32 | 0 | 5 |
| B | 40 | 100 | 0 |
| | 8 | 78 | 1 |
| | 1.6 | 0 | 3 |
| | 0.32 | 0 | 5 |
| Untreated | | 0 | 5 |

## Claims

1. Use of compounds of the formula (1) 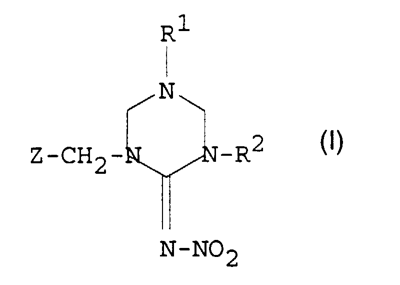 wherein
Z represents 2-chloro-5-pyridyl or 2-chloro-5-thiazolyl, and
R¹ and R² each independently represents hydrogen or C₁₋₄-alkyl, to preserve
wood or composite wood materials against attack from wood destroying insects from the orders isoptera and/or coleoptera.

2. Use according to claim 1 of compounds of formula I
wherein Z represents 2-chloro-5-pyridyl or 2-chloro-5-thiazolyl, and
R¹ and R² represent methyl.

3. A method of preserving wood or composite wood materials against wood destroying insects from the orders isoptera and/or coleoptera which comprise treating wood or composite wood materials with an effective amount of compounds of formula (1) according to claim 1.

## Patentansprüche

1. Verwendung von Verbindungen der Formel (I) 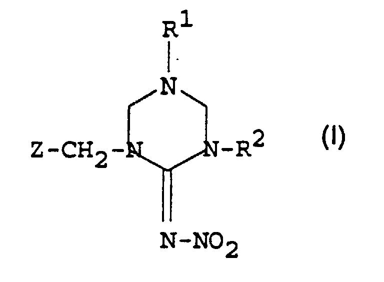 wobei Z 2-Chlor-5-pyridyl oder 2-Chlor-5-thiazolyl darstellt und R¹ und R² jeweils unabhängig Wasserstoff oder C₁₋₄-Alkyl darstellen, zum Schutz von Holz oder Holzverbundmaterialien vor einem Befall durch holzzerstörende Insekten aus den Ordnungen Isoptera und/oder Coleoptera.

2. Verwendung gemäß Anspruch 1 von Verbindungen der Formel I, wobei Z 2-Chlor-5-pyridyl oder 2-Chlor-5-thiazolyl darstellt und R¹ und R² Methyl darstellen.

3. Verfahren zum Schutz von Holz oder Holzverbundmaterialien vor holzzerstörenden Insekten aus den Ordnungen Isoptera und/oder Coleoptera, das das Behandeln von Holz oder Holzverbundmaterialien mit einer wirksamen Menge von Verbindungen der Formel (I) gemäß Anspruch 1 umfaßt.

## Revendications

1. Utilisation d'un composé de formule (I) 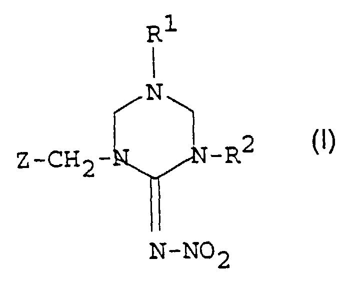 dans laquelle Z représente un radical 2-chloro-5-pyridyle ou 2-chloro-5-thiazolyle et R¹ et R² représentent chacun indépendamment un atome d'hydrogène ou un radical alcoyle en C₁₋₄, pour préserver les matériaux en bois ou en bois composite contre l'attaque par les insectes détruisant le bois de l'ordre des isoptères et/ou des coléoptères.

2. Utilisation selon la revendication 1 des composés de formule I dans laquelle Z représente un radical 2-chloro-5-pyridyle ou 2-chloro-5-thiazolyle et R¹ et R² représentent un radical méthyle.

3. Procédé de préservation des matériaux en bois ou en bois composite contre les insectes détruisant le bois de l'ordre des isoptères et/ou des coléoptères qui comprend le traitement des matériaux en bois ou en bois composite avec une quantité efficace des composés de formule I selon la revendication 1.
